# EUROPÄISCHE PATENTSCHRIFT

(11) **EP 3 175 929 B1**
(45) Veröffentlichungstag und Bekanntmachung des Hinweises auf die Patenterteilung: **20.06.2018**
(21) Anmeldenummer: 17153370.6
(22) Anmeldetag: 01.10.2012
(51) Int. Cl.: B05C 17/01, A61B 17/88, B05C 17/005

(54) **AUSTRAGVORRICHTUNG FÜR HALBFESTE MATERIALIEN**
DISCHARGE DEVICE FOR SEMI-SOLID MATERIALS
DISPOSITIF DE DISTRIBUTION DE MATÉRIAUX SEMI-SOLIDES

(30) Priorität: 01.11.2011 CH 17582011
(43) Veröffentlichungstag der Anmeldung: 07.06.2017
(62) Teilanmeldung aus: 12775599.9
(73) Patentinhaber: Medmix Systems AG, 6343 Rotkreuz (CH)
(72) Erfinder: GRETER, Andy, 6648 Minusio (CH); MATHYS, Beat, 5630 Muri (CH)
(74) Vertreter: Detken, Andreas

(56) Entgegenhaltungen:
- EP-A1- 0 412 198
- DE-U1- 29 821 422
- GB-A- 879 272
- US-A- 2 102 939
- US-A- 4 738 664
- US-A- 5 692 642
- US-A- 5 743 431
- US-A1- 2005 070 912
- US-A1- 2011 218 513

## Beschreibung

### TECHNISCHES GEBIET

Die vorliegende Erfindung betrifft eine Austragvorrichtung. Die erfindungsgemässe Austragvorrichtung eignet sich insbesondere zum Austragen von halbfesten Materialien wie Zementen (insbesondere Knochenzementen oder dentalen Zementen) oder künstlichen Knochenersatzmaterialien, aber auch für andere Arten von fliessfähigen Materialien, insbesondere für alle Arten von hochviskosen, zähflüssigen Materialien.

### STAND DER TECHNIK

Zum Austragen von hochviskosen Fluiden sind sogenannte Pistolendispenser bekannt. Ein derartiger Dispenser weist üblicherweise einen Grundkörper auf, an dem eine Kartusche oder Spritze mit dem darin aufgenommenen Fluid gehalten ist. Mit dem Grundkörper sind ein starrer Handgriff und ein schwenkbarer Betätigungshebel verbunden. Durch den Grundkörper hindurch erstreckt sich eine Kolbenstange. Wenn der Benutzer den Betätigungshebel aus seiner Ausgangsstellung in Richtung des Handgriffs zieht, wird die Kolbenstange gegenüber dem Grundkörper in eine distale Richtung vorgeschoben. Die Kraftübertagung zwischen dem Betätigungshebel und der Kolbenstange kann dabei direkt oder indirekt erfolgen. Der Betätigungshebel ist derart federbelastet, dass er beim Loslassen in seine Ausgangsstellung zurückkehrt. Bei einer erneuten Betätigung des Betätigungshebels erfolgt ein weiterer Vorschub der Kolbenstange.

Aus der US 2007/0289998 A1 ist eine Austragvorrichtung bekannt, bei der eine Mehrkomponentenspritze oder -kartusche mit ihrem Halteflansch seitlich in eine Spritzenhalterung einschiebbar ist. Bei dieser Vorrichtung ist es möglich, je nach Spritzenart und -grösse eine unterschiedliche Spritzenhalterung anzufertigen. Dabei können die Spritzenhalterungen gegebenenfalls auswechselbar sein. Die Vorrichtung eignet sich für speziell dazu passende Mehrkomponentenspritzen mit passendem Halteflansch, jedoch nicht für andere Arten von Behältern, die keinen Halteflansch aufweisen.

EP 0412198 A1 offenbart ein Auspresswerkzeug zum Auspressen pastöser Massen aus einer Kartusche. Das Auspresswerkzeug weist in einem Gehäuse einen Vorschubmechanismus oder eine pneumatische Drückvorrichtung auf. Die Kartusche wird in das rückwärtige Ende eines Kartuschenhalters eingeschoben, und dieser wird mit einem Schnellverschluss am Gehäuse befestigt. Für Kartuschen unterschiedliche Grössen und Formen braucht lediglich ein entsprechend angepasster Kartuschenhalter verwendet zu werden.

US 4,738,664 offenbart einen Pistolendispenser mit einem abnehmbaren Fluidbehälter. Der Behälter wird von einem Kragen abgenommen, in dem er um eine Vierteldrehung oder weniger gegenüber dem Kragen verdreht wird. Der Kragen ist in einem Rahmen eingeschraubt.

GB 879,272 offenbart ebenfalls einen Pistolendispenser. Dieser weist ein Rohr auf, das an einem Ring mit Innengewinde befestigt ist. In das Rohr kann eine Kartusche mit Dichtmasse eingesetzt werden. Ein ähnlich aufgebaute Pistolendispenser ist auch in US 2,102,939 offenbart.

DE 298 21 422 U1 offenbart eine Spritze zum Applizieren von fliessfähigen Produkten. Diese weist einen Adaptereinsatz auf, der als axial geteilte Halbschale ausgebildet ist, in welcher eine Aufnahme für eine Kapsel oder Kartusche ausgedreht ist.

US 5,743,431 offenbart einen Adapter für einen Pistolendispenser. Der Adapter bildet einen Kartuschenhalter mit einer Aufnahme für eine auswechselbare Kartusche sowie eine Aktivierungskammer. Der Kartuschenhalter lässt sich abnehmbar an einem Pistolendispenser befestigen.

Um synthetische Knochenersatzmaterialien auszutragen, ist es bekannt, derartige Materialien in einem zylindrischen Rohr zu verpacken, welches einen im Wesentlichen konstanten Innendurchmesser aufweist und welches sich insbesondere auch an seinem Auslassende nicht verjüngt. Auf diese Weise können ein übermässiger Gegendruck und Verstopfungen beim Austragen verhindert werden. Auch wurde schon vorgeschlagen, Knochenersatzmaterialien aus einem solchen Rohr mit einem Pistolendispenser auszutragen. Ein solches System wird z.B. von Baxter International Inc. unter der Bezeichnung Actifuse™ MIS System angeboten. Am proximalen Ende ist das Rohr mit einem Bajonettanschluss versehen und mit einem verschiebbaren Kolben verschlossen, während das Rohr am distalen Ende mit einer aufschiebbaren Kappe verschlossen ist. Um das Knochenersatzmaterial zu verabreichen, befestigt der Chirurg das Rohr über den Bajonettanschluss am Dispenser und entfernt die Kappe vom distalen Ende. Die Handhabung eines solchen Systems ist für den Chirurgen nicht besonders intuitiv. Da das Rohr bei dem bekannten System mehr als 20 cm lang ist, kann es für den Chirurgen zudem schwierig sein, mit einem solchen System schwer zugängliche Stellen zu erreichen.

### DARSTELLUNG DER ERFINDUNG

Es ist eine Aufgabe der vorliegenden Erfindung, eine Austragvorrichtung anzugeben, die es ermöglicht, auf einfache Weise unterschiedliche Behälter an der Austragvorrichtung anzuschliessen, ohne die Mechanik der Vorrichtung verändern zu müssen. Diese Aufgabe wird durch die Merkmale des Anspruchs 1 gelöst und auch die Merkmale des Anspruchs 3 gelöst. Weitere Ausführungsformen sind in den abhängigen Ansprüchen angegeben.

Es wird also eine Austragvorrichtung angegeben, die einen Grundkörper, einen gegenüber dem Grundkörper von Hand verschwenkbaren Betätigungshebel und eine gegenüber dem Grundkörper verschiebbare Kolbenstange umfasst. Die Kolbenstange ist derart mit dem Betätigungshebel gekoppelt, dass eine Betätigung des Betätigungshebels einen Vorschub der Kolbenstange in eine distale Vorschubrichtung bewirkt. Um auf einfache Weise unterschiedliche Behälter anschliessen zu können, umfasst die Austragvorrichtung einen Adapter, der separat vom Grundkörper ausgebildet ist, der am Grundkörper befestigt ist, und an dem eine Befestigungsstruktur zur Anbringung eines Behälters im Wesentlichen entlang einer entgegen der Vorschubrichtung weisenden proximalen Richtung ausgebildet ist.

Anders als die Vorrichtung der US 2007/0289998 ermöglicht es eine solche Vorrichtung, andere Arten von Behältern, die im Gegensatz zu Spritzen keinen Halteflansch aufweisen, mit der Austragvorrichtung zu verbinden. Insbesondere ermöglicht es diese Vorrichtung, einen Behälter im Wesentlichen entgegen der Vorschubrichtung anzuschliessen, z.B. über eine Schraub-, Bajonett- oder axiale Rastverbindung. Dazu kann die Befestigungsstruktur z.B. ein in die Vorschubrichtung offener Schraubanschluss, Bajonettanschluss oder eine entsprechende Rastaufnahme für den Behälter sein.

Bei dem anzuschliessenden Behälter kann es sich um einen Behälter in Form eines (geraden oder gekrümmten) zylindrischen Rohrs mit im Wesentlichen konstantem Innendurchmesser handeln, wie es unten noch näher beschrieben ist. Es können jedoch auch ganz andere Arten von Behältern angeschlossen werden, die sich unter Umständen am distalen Ende auch verjüngen können. Beim anzuschliessenden Behälter handelt es sich bevorzugt um einen Einzelbehälter mit einem einzigen Reservoir, in dem ein Kolben verschiebbar ist, an welchem wiederum die Kolbenstange angreift, also nicht um einen Mehrfachbehälter mit mehreren parallel angeordneten Reservoirs. Eine Nutzung einer solchen Vorrichtung mit Mehrfachbehältern ist jedoch nicht ausgeschlossen.

Vorzugsweise weist die Vorrichtung die Form eines Pistolendispensers auf, d.h. sie umfasst einen Handgriff, der starr am Grundkörper ausgebildet oder angebracht ist, und der Betätigungshebel wird auf den Handgriff zu bewegt, um die Kolbenstange vorzuschieben. In bevorzugten Ausführungsformen wird dazu der Handgriff mit einer Hand eines Benutzers ergriffen, und der Betätigungshebel wird mit den Fingern derselben Hand im Wesentlichen entgegen der Vorschubrichtung der Kolbenstange an den Handgriff herangezogen. Der Betätigungshebel kann dazu nahe seinem oberen oder unteren Ende mit dem Handgriff schwenkbar verbunden sein, oder er kann durch eine Verschiebung auf den Handgriff zu bewegbar sein. Pistolendispenser sind in einer Vielzahl von Ausgestaltungen aus dem Stand der Technik bekannt, und die vorliegende Erfindung ist in keiner Weise auf eine bestimmte Art von Pistolendispenser beschränkt. Beispielhaft sei hier auf die Dokumente US 5,137,181, US 5,464,131, US 5,992,694, US 2007/0289998 oder WO 2011/009221 verwiesen, die jeweils Pistolendispenser zeigen, deren mechanischer Aufbau zur Bewegungsübertragung vom Betätigungshebel auf die Kolbenstange im Zusammenhang mit der vorliegenden Erfindung eingesetzt werden kann. Es können aber auch andere Bauformen von Dispensern zum Einsatz kommen, bei denen ein Hebel eine Vorschubbewegung einer Kolbenstange erzeugt, z.B. Dispenser, in denen der bewegliche Hebel durch den Handballen des Benutzers auf den Handgriff zu bewegt wird, wie dies z.B. in US 4,744,494 oder CH 641 736 dargestellt ist, oder Stiftdispenser wie in US 5,735,437 oder WO 2005/084819.

Gemäss einer ersten Ausführungsform ist der Adapter abnehmbar am Grundkörper befestigt. Um den Adapter einfach montieren zu können, ist der Adapter in diesem Fall entlang einer quer zur Vorschubrichtung verlaufenden Einschubrichtung in den Grundkörper einschiebbar. Insbesondere ist es bevorzugt, dass der Adapter seitlich in den Grundkörper einschiebbar ist, d.h. die Einschubrichtung verläuft vorzugsweise quer zu einer Ebene, die durch die Kolbenstange und den Betätigungshebel bzw. durch den Handgriff und den Betätigungshebel definiert ist. Der Adapter kann aber auch z.B. von oben her in den Grundkörper einschiebbar sein. Der Begriff "quer" ist dabei im vorliegenden Dokument wie folgt zu verstehen: Eine Richtung verläuft "quer" zu einer anderen Richtung, wenn der Winkel zwischen den Richtungen zwischen 45° und 135°, vorzugsweise zwischen 60° und 120°, insbesondere zwischen 75° und 105° und bevorzugt ungefähr 90° beträgt.

Der Adapter kann insbesondere die folgende Form aufweisen: Er kann einen Hauptabschnitt mit einer axialen Durchgangsöffnung für die Kolbenstange, eine am Hauptabschnitt ausgebildete Befestigungsstruktur für den Behälter, die sich vorzugsweise mindestens teilweise um die Durchgangsöffnung herum erstreckt (z.B. eine Gewindebuchse, die sich um die Durchgangsöffnung herum erstreckt, oder eine Bajonettaufnahme) sowie zwei sich gegenüberliegende und sich im Wesentlichen in entgegengesetzte laterale Richtungen erstreckende Halteflügel aufweisen. Der Grundkörper und die Halteflügel können in ihrer Form dabei im Wesentlichen der Form des proximalen Endes eines an sich bekannten Spritzenbehälters entsprechen, d.h. der Adapter kann in einen Grundkörper einschiebbar sein, der an sich dazu ausgebildet ist, eine (Einfach- oder Mehrfach-) Spritze aufzunehmen. Indem der Adapter die Form einer Spritze aufgreift, ermöglicht es der Adapter, einen Dispenser, der an und für sich für die Verwendung mit Spritzen ausgebildet ist, auch mit anderen Arten von Behältern zu verwenden. Der Grundkörper der Austragvorrichtung wird in diesem Fall eine zum Adapter komplementäre Einschuböffnung mit zwei sich gegenüberliegenden und sich quer zur Vorschubrichtung und quer zur Einschubrichtung erstreckenden Schlitzen zur Aufnahme der Halteflügel aufweisen. Insbesondere kann die Einschuböffnung wie in der schon erwähnten US 2007/0289998 ausgebildet sein.

Gemäss einer alternativen Ausgestaltung weist der Grundkörper ein Gehäuse sowie ein separat gefertigtes und mit dem Gehäuse verbundenes, insbesondere abnehmbares Halteelement auf, wobei der Adapter am Halteelement angebracht ist. Nicht nur der Adapter ist in diesem Fall also leicht durch einen anderen Adapter ersetzbar, sondern auch das Halteelement, das zur Verbindung des Adapters mit dem Grundkörper dient, kann bei der Herstellung oder später auf einfache Weise gegen ein anderes Halteelement ausgetauscht werden. Dadurch wird eine noch grössere Flexibilität bei der Art der anzuschliessenden Behälter ermöglicht. So können ohne konstruktive Änderungen der Hebelmechanik z.B. Dispenser für verschiedene Grössen von Spritzen (die direkt mit dem Halteelement verbunden werden) als auch für verschiedene Arten von Schraubbehältern (für die jeweils ein Adapter zum Einschub in ein bestimmtes Halteelement vorgesehen ist) realisiert werden. Da die konstruktiv aufwändige Hebelmechanik für alle diese Dispenser immer identisch ist, kann eine ganze Serie von Dispensern nach Art eines Baukastensystems geschaffen werden, bei der die kostenintensivsten Teile immer identisch konstruiert sind und daher in grosser Stückzahl kostengünstig produziert werden können.

Das Halteelement kann wiederum entlang einer quer zur Vorschubrichtung verlaufenden Befestigungsrichtung in das Gehäuse einschiebbar sein. Dabei kann die Befestigungsrichtung mit der Einschubrichtung für den Adapter identisch sein oder auch z.B. quer zu dieser Richtung verlaufen.

Vorzugsweise kann der Adapter ausgetauscht werden, ohne die Kolbenstange vollständig aus dem Grundkörper zu entfernen. Der Adapter ist also vorzugsweise vom Grundkörper abnehmbar, während die Kolbenstange am Grundkörper gehalten ist. Dadurch wird der Austausch des Adapters erleichtert. Im Gegensatz hierzu ist der Spritzenhalter der US 2007/0289998 nur austauschbar, wenn die Kolbenstange vollständig entfernt wurde.

die Austragvorrichtung ist mit einem Behälter verwendbar, wobei der Behälter mit einem hochviskosen oder halbfesten, vorzugsweise implantierbaren Material gefüllt ist, z.B. mit einem synthetischen Knochenersatzmaterial (einem "bone graft substitute"). Der Behälter weist die Form eines zylindrischen Rohres auf, welches über seine gesamte Länge hinweg eine im Wesentlichen konstante Querschnittsfläche (und somit einen konstanten Innendurchmesser) aufweist und welches an seinen beiden Enden jeweils eine aussenseitige Verbindungsstruktur aufweist. Die Verbindungsstrukturen sind dabei an beiden Enden gleichartig ausgebildet. Dadurch ist es möglich, den Behälter mit jedem seiner beiden Enden an der Austragvorrichtung zu befestigen. Vorzugsweise sind die Verbindungsstrukturen an beiden Enden sogar im Wesentlichen identisch. Auf diese Weise wird eine besonders intuitive Handhabung gewährleistet. Der Behälter ist zudem vorzugsweise beidseitig mit jeweils einer Verschlusskappe verschlossen, die mit den Verbindungsstrukturen in Eingriff steht. Die Verschlusskappen sind vorzugsweise im Wesentlichen identisch.

Insbesondere können an beiden Enden Aussengewinde vorgesehen sein. Auf jedes Ende ist dann vorzugsweise jeweils eine Verschlusskappe mit einem Innengewinde entfernbar aufgeschraubt. Dadurch wird eine gute Dichtigkeit gewährleistet. Es ist aber auch denkbar, an beiden Enden andere Arten von Verbindungsstrukturen, z.B. aussenseitige Zapfen, aussenseitige Stege und/oder aussen eingelassene Nuten für eine Bajonettverbindung vorzusehen. Auch in diesem Fall kann auf beide Enden eine entsprechend komplementär ausgebildete Verschlusskappe aufgesetzt sein. Wenn Aussengewinde vorhanden sind, weisen diese bevorzugt an beiden Enden des Behälters identische Kenngrössen, insbesondere Zahl der Gänge, Flankenform, Aussen- und Kerndurchmesser und Steigung auf. Vorzugsweise sind die Gewinde und bevorzugt auch die Verschlusskappen zudem auch noch in jeder anderen Hinsicht identisch (weisen also insbesondere auch die gleiche Länge auf). Auf diese Weise wird für den Benutzer sofort offensichtlich, dass es auf die Orientierung des Behälters gar nicht ankommt. Die Anbringung des Behälters wird dadurch besonders intuitiv. Dies erleichtert dem Benutzer (in der Regel ein Chirurg) die Arbeit und reduziert das Risiko von Fehlbedienungen. Dies kann insbesondere in der Hektik eines Operationssaales ein wichtiger Faktor sein. Ähnliches gilt auch für andere Arten von Verbindungsstrukturen und Verschlusskappen.

Der Behälter enthält bevorzugt vor dem erstmaligen Entfernen der Verschlusskappen noch keinen verschiebbaren Kolben, d.h. der Kolben wird in diesem Fall erst durch die Austragvorrichtung bereitgestellt. Dies verringert die Gefahr, dass der Kolben durch längere Lagerung im Behälter festsitzt, schafft aber auch die Möglichkeit, den Behälter überhaupt in einer beliebigen Orientierung an der Austragvorrichtung anzuschliessen.

Der Behälter weist vorzugsweise eine Länge auf, die mindestens das Fünffache, bevorzugt mindestens das Zehnfache, besonders bevorzugt mindestens das 20-fache des Innendurchmessers beträgt. In absoluten Zahlen beträgt die Länge des Rohres vorzugsweise mindestens 100 mm, bevorzugt ca. 150-300 mm. Der Innendurchmesser des Rohres beträgt bevorzugt ca. 3-10 mm, besonders bevorzugt ca. 5-7 mm. Die Wandstärke des Rohres beträgt vorzugsweise ca. 0.5-1 mm. Das Rohr kann z.B. aus Polypropylen (PP), Polyamid (PA), Polyetheretherketon (PEEK) oder Polycarbonat (PC) bestehen, während die Verschlusskappen aus demselben oder auch aus einem anderen Material gefertigt sein können.

Der Behälter kann nicht nur ein gerades Rohr bilden, sondern kann auch die Form eines gekrümmten Rohres aufweisen, so dass sein distales Ende mit dem proximalen Ende einen Winkel von mindestens 15°, bevorzugt mindestens 30° oder sogar 45° einschliesst, oder derartig biegsam sein, dass sein distales Ende gegenüber dem proximalen Ende schwenkbar ist, insbesondere um einen Winkel von mindestens 45° schwenkbar ist. Dadurch wird es möglich, auch schwer zugängliche Stellen zu erreichen. Ein flexibler Behälter kann z.B. aus einem flexiblen Polyethylen (PE), aus PP oder aus Polytetrafluorethylen (PTFE) hergestellt sein. Wenn der Behälter biegsam ist, ist es bevorzugt, dass das Biegemoment weniger als 2.0 Nm, besonders bevorzugt weniger als 1.0 Nm, besonders bevorzugt weniger als 0.5 Nm oder sogar weniger al 0.1 Nm beträgt, wenn der Behälter zu einem Krümmungsradius von 100 mm gebogen wird.

Um die Vorrichtung auch mit gekrümmten oder flexiblen Behältern, wie z.B. den oben beschriebenen gekrümmten oder flexiblen Behältern, verwendbar zu machen und so die Erreichbarkeit schwer zugänglicher Stellen zu verbessern, kann die Kolbenstange derart biegsam sein, dass ihr distales Ende um einen Winkel von mindestens 45°, vorzugsweise mindestens 60°, weiter bevorzugt mindestens 90°, besonders bevorzugt mindestens 135° oder sogar mindestens 180° gegenüber ihrem proximalen Ende biegbar ist, ohne dass die Kolbenstange bricht.

Die Biegung kann dabei entlang einer einzigen Biegerichtung möglich sein, oder die Kolbenstange kann sogar um zwei oder mehr Biegerichtungen biegsam sein. Die Kolbenstange ist so ausgestaltet, dass das Biegemoment entlang mindestens einer Richtung vorzugsweise weniger als 2.0 Nm, besonders bevorzugt weniger als 1.0 Nm, besonders bevorzugt weniger als 0.5 Nm oder sogar weniger als 0.1 Nm beträgt, wenn die Kolbenstange zu einem Krümmungsradius von 100 mm gebogen wird.

Um die Biegsamkeit zu verbessern, kann die Kolbenstange eine erste Seite aufweisen, auf der eine Mehrzahl von Zähnen mit regelmässigem Abstand ausgebildet sind, wobei der Betätigungshebel bei seiner Betätigung direkt oder indirekt eine Vorschubkraft auf die Zähne ausübt, um den Vorschub der Kolbenstange zu bewirken, und wobei die Kolbenstange mindestens auf einer der ersten lateralen Seite gegenüberliegenden zweiten Seite eine Vielzahl von Einschnitten aufweist, um die Biegsamkeit der Kolbenstange zu erhöhen. Die Einschnitte können gerade oder z.B. keilförmig sein. Sie können einen Abstand voneinander aufweisen, der dem Zahnabstand oder einem Mehrfachen davon entspricht. Bevorzugt liegt jeweils einem Zahngrund ein Einschnitt gegenüber, so dass in diesem Bereich die Materialstärke von beiden Seiten her verringert ist. Die Materialstärke kann dabei so gering sein, dass zwischen benachbarten Zähnen ein eigentliches Filmscharnier ausgebildet ist. Zusätzlich können solche Einschnitte auch an weiteren Seiten der Kolbenstange vorgesehen sein, z.B. an denjenigen Seiten, die gegenüber den Zähnen um 90° um die Vorschubachse versetzt angeordnet sind. Selbstverständlich sind aber auch völlig andere Ausgestaltungen der Kolbenstange möglich. So können z.B. auch richtiggehende Gelenke in der Kolbenstange ausgebildet sein.

Eine solche Vorrichtung wird vorzugsweise zusammen mit einem Behälter in Form eines zylindrischen Rohres verwendet, welches gekrümmt ist, so dass sein distales Ende mit dem proximalen Ende einen Winkel von mindestens 15°, bevorzugt mindestens 30° oder sogar 45° einschliesst, oder welches derartig biegsam ist, dass sein distales Ende gegenüber dem proximalen Ende um einen Winkel von mindestens 45° schwenkbar ist. Dabei sind die Länge der Kolbenstange und des Rohres so gewählt, dass sich die Kolbenstange im Behälter im Wesentlichen bis ans distale Ende des Behälters vorschieben lässt. Für die weiteren Eigenschaften wie Biegsamkeit, Materialwahl usw. gelten sinngemäss die oben ausgeführten Überlegungen zum erfindungsgemässen Behälter, auch falls der verwendete Behälter keine oder andere Verbindungsstrukturen als oben angegeben aufweisen sollte.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: ein bevorzugtes Ausführungsbeispiel einer erfindungsgemässen Austragvorrichtung mit angeschlossenem Behälter in einer isometrischen Ansicht;
- Fig. 2: eine Teilansicht der Austragvorrichtung der Fig. 1 ohne Behälter, ohne Adapter, ohne Halteelement und ohne Abdeckung;
- Fig. 3: eine Explosionsdarstellung der Austragvorrichtung der Fig. 1 ohne Behälter;
- Fig. 4: eine Ansicht der Austragvorrichtung der Fig. 1 mit angeschlossenem Behälter, bei der der Behälter teilweise transparent dargestellt ist;
- Fig. 5: ein Kit mit der Austragvorrichtung der Fig. 1 und mehreren zugehörigen Behältern;
- Fig. 6: die Austragvorrichtung der Fig. 1 zusammen mit unterschiedlich geformten Behältern; und
- Fig. 7: die Austragvorrichtung der Fig. 1 zusammen mit unterschiedlichen Arten von Behältern.

### BESCHREIBUNG BEVORZUGTER AUSFÜHRUNGSFORMEN

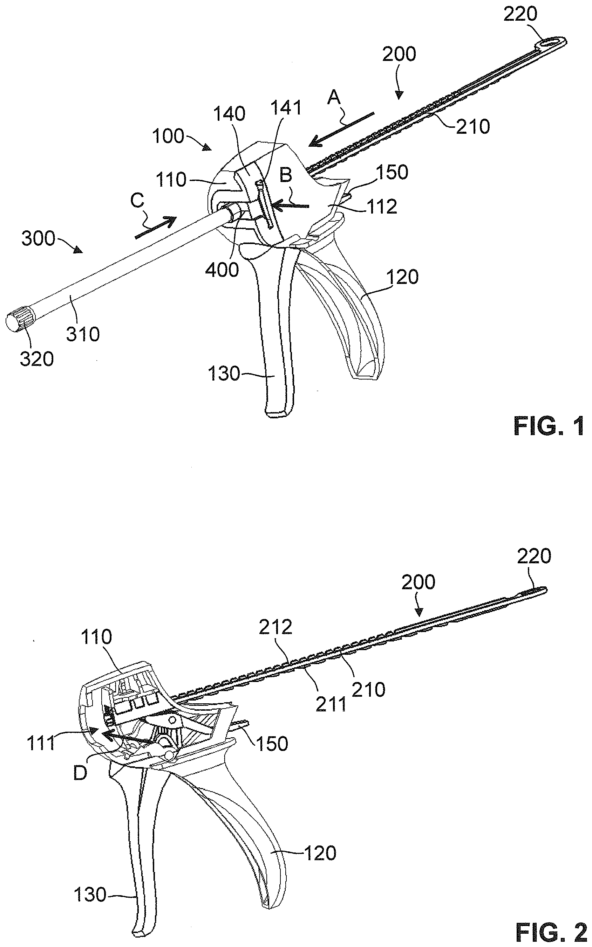

In den Figuren 1-4 ist in unterschiedlichen Ansichten eine Austragvorrichtung 100 in Form eines Pistolendispensers illustriert. Die Vorrichtung umfasst einen Grundkörper 110, an dem einstückig ein starrer Handgriff 120 ausgebildet ist. Ein Betätigungshebel 130 ist schwenkbar mit dem Grundkörper 110 verbunden. Über einen nicht im Detail dargestellten Mechanismus ist der Betätigungshebel 130 mit einer Kolbenstange 200 gekoppelt, die sich durch den Grundkörper 110 hindurch erstreckt. Durch Betätigung des Betätigungshebels 130 ist die Kolbenstange 200 entlang einer distalen Vorschubrichtung A gegenüber dem Grundkörper 110 verschiebbar. Dazu sind auf der Unterseite der Kolbenstange eine Vielzahl von Zähnen 211 (siehe Fig. 2) ausgebildet. Der betreffende Mechanismus kann z.B. entsprechend der US 5,992,694 ausgebildet sein. Eine Rücklaufsperre verhindert ein unbeabsichtigtes Zurückziehen der Kolbenstange entgegen der Vorschubrichtung A. Diese Sperre kann durch Betätigung eines Freigabehebels 150 gelöst werden.

Ein Halteelement 140 ist seitlich entlang einer Befestigungsrichtung D in eine Befestigungsöffnung 111 im Gehäuse des Grundkörpers 110 eingeschoben (siehe Fig. 2) und dort durch eine Abdeckung 112 (siehe Fig. 1) fixiert. Das Halteelement 140 ist als vereinzeltes Austauschteil ausgebildet und kann je nach konkreten Anforderungen bei der Produktion oder, wenn das Halteelement lösbar angebracht ist, auch nachträglich leicht durch ein anders geformtes Halteelement ersetzt werden, ohne die Mechanik der Vorrichtung zu verändern. Das Halteelement definiert eine seitlich offene Einschuböffnung 141. Diese weist zwei sich gegenüberliegende Schlitze 142, 143 auf, die sich nach unten bzw. nach oben erstrecken, d.h. quer zur Vorschubrichtung A und quer zur Befestigungsrichtung D. In einem zentralen Bereich zwischen diesen Schlitzen ist die Einschuböffnung auch in die distale Richtung offen.

Insoweit entspricht der Aufbau der Austragvorrichtung weitgehend dem Aufbau des Austraggeräts der US 2007/0289998, deren Inhalt durch Verweis in dieses Dokument aufgenommen wird, soweit dort der grundsätzliche Aufbau des in den Figuren 1-4 dargestellten Austraggeräts offenbart ist.

Am distalen Ende der Kolbenstange 200 ist ein Kolben 230 ausgebildet; am proximalen Ende ist ein flacher Handgriff 220 ausgebildet. Dazwischen erstreckt sich der ebenfalls abgeflacht ausgebildete Hauptabschnitt 210 der Kolbenstange. Die Kolbenstange 200 lässt sich mit ihrem proximalen Ende axial entgegen der Vorschubrichtung A durch die Einschuböffnung 141 hindurch in die Vorrichtung einschieben, während der Freigabehebel 150 gedrückt wird. Beim Zurückziehen der Kolbenstange 200 bildet der Kolben 230 einen Anschlag, über den hinaus die Kolbenstange nicht weiter zurückgezogen werden kann.

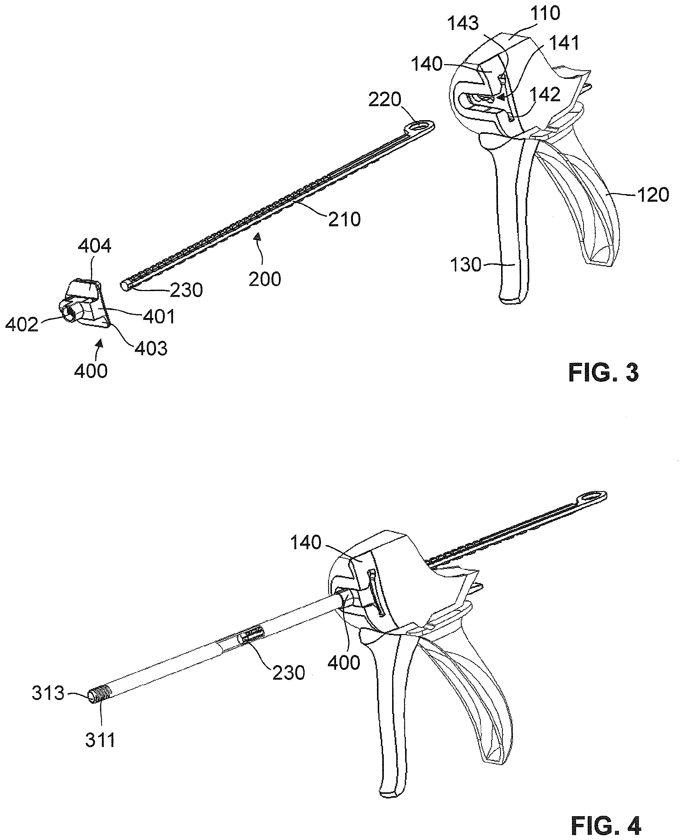

In die Einschuböffnung 141 ist entlang einer Einschubrichtung B seitlich ein Adapter 400 eingeschoben, der in der Fig. 3 besonders gut erkennbar ist. Die Einschubrichtung B verläuft entlang einer Richtung, die quer zur Ebene verläuft, welche durch den Handgriff 120 und den Hebel 130 definiert ist und welche auch die Kolbenstange 200 entlang ihrer gesamten Länge schneidet. Im vorliegenden Beispiel entspricht die Einschubrichtung gleichzeitig der Befestigungsrichtung D, entlang derer das Halteelement 140 in das Gehäuse des Grundkörpers 110 eingeschoben ist, sie kann aber in anderen Ausgestaltungen des Halteelements 140 auch anders gewählt sein.

Der Adapter 400 entspricht in seiner äusseren Form in etwa dem hinteren Ende der Doppelspritze der US 2007/0289998. Er weist einen hier im Wesentlichen quaderförmig ausgebildeten Hauptabschnitt 401 auf, durch den hindurch eine Durchgangsöffnung zur Durchführung der Kolbenstange 200 ausgebildet ist. An den Hauptabschnitt schliesst sich in axialer, distaler Richtung eine ringförmige Befestigungsstruktur 402 an, die die Durchgangsöffnung radial umgibt und an deren Innenseite ein Innengewinde ausgebildet ist. Vom Hauptabschnitt aus erstrecken sich zwei Halteflügel 403, 404 nach unten bzw. nach oben, also lateral, d.h. quer zur axialen Richtung.

Die Form des Adapters 400 ist komplementär zur Form der Einschuböffnung 141 gewählt. Dadurch lässt sich der Adapter 400 ohne weiteres anstelle einer Doppelspritze seitlich in die Einschuböffnung 141 einschieben. Dabei kann die Kolbenstange 200 in der Vorrichtung verbleiben; sie muss lediglich bis zu ihrem Anschlag in die proximale Richtung zurückgezogen werden. Montage oder Austausch des Adapters können dadurch sehr einfach erfolgen.

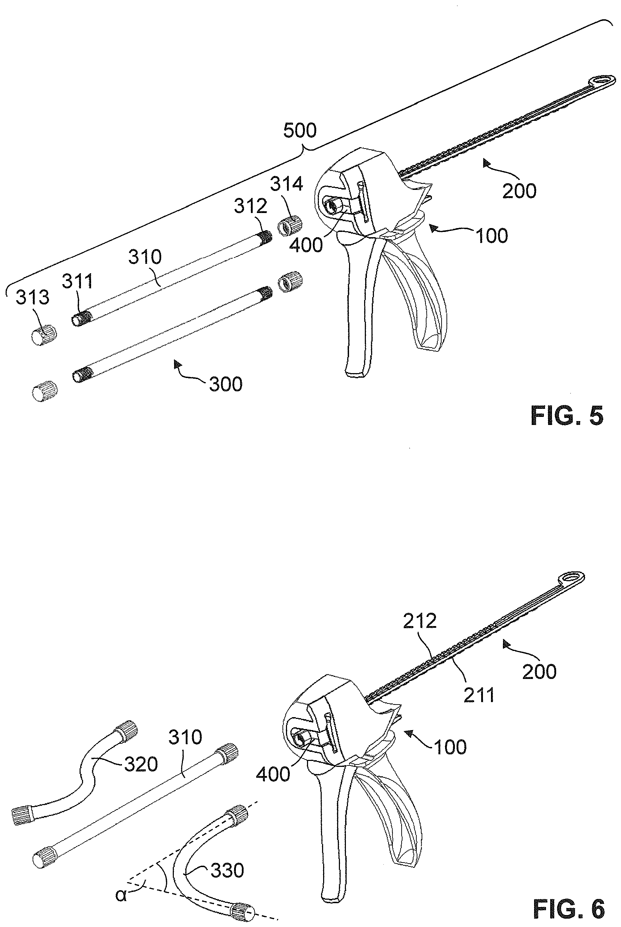

In die Befestigungsstruktur 402 des Adapters 400 ist ein Behälter 300 eingeschraubt, der in der Fig. 5 näher dargestellt ist. Der Behälter weist in Form eines geraden zylindrischen Rohrs 310 auf. Das Rohr hat innen einen zylindrischen Querschnitt, der sich über seine gesamte Länge nicht wesentlich verändert und der sich insbesondere auch nicht an den Enden des Rohrs nicht verjüngt. Das Rohr 310 ist mit einem nur schwer fliessfähigen, implantierbaren Material, z.B. einem synthetischen Knochenersatzmaterial oder einem Zement, gefüllt. An beiden Enden weist das Rohr identische Aussengewinde 311, 312 auf. Vor dem Einsetzen in die Austragvorrichtung ist das Rohr an beiden Enden durch identische Verschlusskappen 313, 314 verschlossen, die ein Innengewinde aufweisen und mit diesem auf die Aussengewinde 311, 312 aufgeschraubt sind. Im Behälter 300 ist dabei kein separater verschiebbarer Kolben aufgenommen.

Die Vorrichtung wird wie folgt bedient. Vor dem Einsetzen des Rohres 310 in die Vorrichtung werden die Verschlusskappen 313, 314 durch den Chirurgen entfernt. Das Rohr 310 wird dann entlang einer proximalen Richtung C, die entgegen der Vorschubrichtung A weist, in die Befestigungsstruktur 402 eingeführt und mittels seines Aussengewindes 311 oder 312 darin eingeschraubt. Dabei kommt es auf die Orientierung des Rohres nicht an, d.h. es spielt keine Rolle, ob das Rohr mit dem Ende, an dem sich das Gewinde 311 befindet, oder mit dem Ende, an dem sich das Gewinde 312 befindet, eingeschraubt wird. Dies erleichtert dem Chirurgen die Handhabung des Behälters. Anschliessend ergreift der Chirurg die Vorrichtung am Handgriff 120 und zieht den Betätigungshebel 130 mit den Fingern derselben Hand zum Handgriff 120 hin. Dadurch wird die Kolbenstange 200 entlang der Vorschubrichtung A vorgeschoben, und der Kolben 230 gelangt ins Innere des Rohrs 310. Die Abmessungen des Kolbens 230 sind dabei so gewählt, dass der Kolben 230 dichtend innen an der Wand des Rohres 310 anliegt. Dadurch presst der Kolben das im Rohr befindliche Material in die distale Richtung und trägt es aus. In der Fig. 4 ist die Vorrichtung in einer Stellung illustriert, in der der Kolben um knapp die Hälfte der Länge des Rohres vorgeschoben wurde.

Die Vorrichtung kann zusammen mit einem oder mehreren Behältern 300 als Set verkauft werden. Ein solches Set 500 mit zwei identischen Behältern 300 ist in der Fig. 5 illustriert.

In der Fig. 6 sind unterschiedlich geformte Behälter illustriert. Während einer der Behälter wie zuvor die Form eines geraden Rohres 310 aufweist, weisen die anderen Behälter die Form eines gekrümmten Rohres 320, 330 auf. Dabei schliessen die Richtungen, die durch die Enden des Rohres definiert werden, einen Winkel α ein, der ohne weiteres 45° oder mehr betragen kann; im vorliegenden Beispiel beträgt der Winkel α für das Rohr 330 ca. 90°. Mit solchen gekrümmten Rohren können auch Stellen erreicht werden, die sonst nicht einfach erreichbar wären. Statt starr gekrümmt kann das Rohr biegsam sein. Damit die Kolbenstange 200 der Krümmung der Rohre 320, 330 folgen kann, ist die Kolbenstange besonders biegsam ausgestaltet. Dazu weist sie auf ihrer Oberseite, gegenüberliegend zu den Zähnen 211, eine Vielzahl von Einschnitten 212 auf, an denen die Materialstärke entsprechend verringert ist.

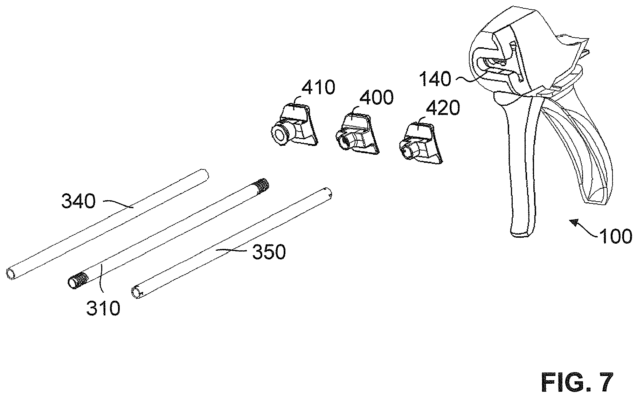

In der Fig. 7 sind unterschiedliche Arten von Behälteranschlüssen und entsprechenden Adaptern illustriert. Statt eines Schraubanschlusses wie bei Rohr 310 und Adapter 400 kann auch eine reine Steckverbindung (Rohr 340 mit Adapter 410) oder eine Bajonettverbindung (Rohr 350 mit Adapter 420) vorgesehen sein. Auch eine axiale Rastverbindung ist denkbar. Indem ein Adapter leicht gegen einen anderen Adapter austauschbar ist, können solche unterschiedlichen Befestigungsarten leicht realisiert werden, ohne die Mechanik der Verabreichungsvorrichtung abändern zu müssen. Der Grundkörper 110 mit der gesamten Mechanik kann dadurch unabhängig von der Art des anzuschliessenden Behälters immer identisch hergestellt werden, was die Produktionskosten gering hält.

Während die Erfindung vorstehend vor allem unter Bezugnahme auf ein bestimmtes medizinisches Anwendungsbeispiel beschrieben wurde, ist die Erfindung keineswegs hierauf beschränkt. Es ist denkbar, die Erfindung auch in völlig anderen, darunter auch nichtmedizinischen Einsatzgebieten zu verwenden, in denen ein nur schwer fliessfähiges Material ausgetragen werden soll.

**BEZUGSZEICHENLISTE**

| | | | |
|---|---|---|---|
| 100 | Austragvorrichtung | 212 | Einschnitte |
| 110 | Grundkörper | 220 | Handgriff |
| 111 | Gehäuse | 230 | Kolben |
| 112 | Abdeckung | 300 | Behälter |
| 120 | Handgriff | 310 | Rohr |
| 130 | Betätigungshebel | 311, 312 | Gewinde |
| 140 | Halteelement | 313, 314 | Verschlusskappe |
| 141 | Einschuböffnung | 320-350 | Rohr |
| 142, 143 | Schlitz | 400 | Adapter |
| 150 | Freigabehebel | 401 | Hauptabschnitt |
| 200 | Kolbenstange | 402 | Befestigungsstruktur |
| 210 | Hauptabschnitt | 403, 404 | Halteflügel |
| 211 | Zähne | 500 | Set |

## Patentansprüche

1. Austragvorrichtung, aufweisend einen Grundkörper (110), einen gegenüber dem Grundkörper (110) von Hand verschwenkbaren Betätigungshebel (130) und eine gegenüber dem Grundkörper (110) verschiebbare Kolbenstange (200), wobei die Kolbenstange (200) derart mit dem Betätigungshebel (130) gekoppelt ist, dass eine Betätigung des Betätigungshebels (130) einen Vorschub der Kolbenstange (200) in eine distale Vorschubrichtung (A) bewirkt, wobei die Austragvorrichtung einen Adapter (400) umfasst, der separat vom Grundkörper (110) ausgebildet und am Grundkörper (110) befestigt ist und an dem eine Befestigungsstruktur zur Anbringung eines Behälters (300) im Wesentlichen entlang einer entgegen der Vorschubrichtung (A) weisenden proximalen Richtung (C) ausgebildet ist, **dadurch gekennzeichnet, dass** der Adapter (400) entlang einer quer zur Vorschubrichtung verlaufenden Einschubrichtung (B) in den Grundkörper (110) einschiebbar ist.

2. Austragvorrichtung nach Anspruch 1, wobei der Adapter (400) einen Hauptabschnitt (401) mit einer Durchgangsöffnung für die Kolbenstange (200), eine am Hauptabschnitt (401) ausgebildete Befestigungsstruktur (402) für den Behälter (300) sowie zwei sich gegenüberliegende und sich in entgegengesetzte laterale Richtungen erstreckende Halteflügel (403, 404) aufweist, und wobei der Grundkörper (110) eine zum Adapter (400) komplementäre Einschuböffnung (141) mit zwei sich gegenüberliegenden und sich quer zur Vorschubrichtung und quer zur Einschubrichtung erstreckenden Schlitzen (142, 143) zur Aufnahme der Halteflügel (403, 404) aufweist.

3. Austragvorrichtung, aufweisend einen Grundkörper (110), einen gegenüber dem Grundkörper (110) von Hand verschwenkbaren Betätigungshebel (130) und eine gegenüber dem Grundkörper (110) verschiebbare Kolbenstange (200), wobei die Kolbenstange (200) derart mit dem Betätigungshebel (130) gekoppelt ist, dass eine Betätigung des Betätigungshebel (130) einen Vorschub der Kolbenstange (200) in eine distale Vorschubrichtung (A) bewirkt, wobei die Austragvorrichtung einen Adapter (400) umfasst, der separat vom Grundkörper (110) ausgebildet und am Grundkörper (110) befestigt ist und an dem eine Befestigungsstruktur zur Anbringung eines Behälters (300) im Wesentlichen entlang einer entgegen der Vorschubrichtung (A) weisenden proximalen Richtung (C) ausgebildet ist, wobei der Grundkörper (110) ein Gehäuse sowie ein separat gefertigtes und mit dem Gehäuse verbundenes Halteelement (140) aufweist, wobei der Adapter (400) lösbar am Halteelement (140) befestigbar ist, **dadurch gekennzeichnet, dass** das Halteelement (140) entlang einer quer zur Vorschubrichtung (A) verlaufenden Befestigungsrichtung (D) in das Gehäuse einschiebbar ist.

4. Austragvorrichtung nach einem der vorhergehenden Ansprüche, wobei der Adapter (400) vom Grundkörper (110) abnehmbar ist, während die Kolbenstange (200) am Grundkörper (110) gehalten ist.

5. Austragvorrichtung nach einem der vorhergehenden Ansprüche, wobei der Adapter (400) einen in die Vorschubrichtung (A) offenen Schraubanschluss oder Bajonettanschluss für den Behälter (300) aufweist.

6. Austragvorrichtung nach einem der vorhergehenden Ansprüche, aufweisend einen Behälter (300), wobei der Behälter (300) mit einem hochviskosen oder halbfesten, vorzugsweise implantierbaren Material gefüllt ist, wobei der Behälter (300) die Form eines zylindrischen Rohres aufweist, welches über seine gesamte Länge hinweg eine im Wesentlichen konstanten Querschnittsfläche aufweist, wobei an beiden Enden des Rohrs jeweils aussenseitig eine Verbindungsstruktur zur Verbindung mit dem Adapter ausgebildet ist, und wobei die Verbindungsstrukturen an beiden Enden gleichartig ausgebildet sind.

7. Austragvorrichtung nach Anspruch 6, wobei die Verbindungsstrukturen an beiden Enden des Behälters (300) im Wesentlichen identisch sind.

8. Austragvorrichtung nach Anspruch 6 oder 7, wobei der Behälter (300) die Form eines gekrümmten Rohres (320; 330) aufweist.

9. Austragvorrichtung nach einem der Ansprüche 6-8, wobei der Behälter (300) derartig biegsam ist, dass sein distales Ende gegenüber dem proximalen Ende um mindestens 45° schwenkbar ist.

10. Austragvorrichtung nach einem der vorhergehenden Ansprüche, wobei die Kolbenstange (200) derart biegsam ist, dass ihr distales Ende entlang mindestens einer Biegerichtung um einen Winkel von mindestens 45° gegenüber ihrem proximalen Ende schwenkbar ist.

11. Austragvorrichtung nach Anspruch 10, wobei die Kolbenstange (200) eine erste Seite aufweist, auf der eine Mehrzahl von Zähnen (211) ausgebildet sind, wobei der Betätigungshebel (130) bei seiner Betätigung direkt oder indirekt eine Vorschubkraft auf die Zähne (211) ausübt, um den Vorschub der Kolbenstange (200) zu bewirken, und wobei die Kolbenstange (200) mindestens auf einer der ersten Seite gegenüberliegenden zweiten Seite eine Vielzahl von Einschnitten (212) aufweist, um die Biegsamkeit der Kolbenstange (200) zu erhöhen.

12. Austragvorrichtung nach Anspruch 10 oder 11, welche ausserdem einen Behälter (300) in Form eines zylindrischen Rohres aufweist, welches gekrümmt ist oder welches derartig biegsam ist, dass sein distales Ende gegenüber dem proximalen Ende um einen Winkel (α) von mindestens 45° schwenkbar ist.

## Claims

1. A discharge device comprising a base body (110), an operating lever (130) that is manually pivotable relative to the base body (110), and a piston rod (200) that is slidable relative to the base body (110), wherein the piston rod (200) is coupled to the operating lever (130) in such a manner that operation of the operating lever (130) causes the piston rod (200) to advance in a distal feed direction (A), wherein the discharge device comprises an adapter (400) that is formed separately from the base body (110) and is attached to the base body (110) and on which a fastening structure is formed for attachment of a container (300) substantially along a proximal direction (C) contrary to the feed direction (A), **characterized in that** the adapter (400) is configured to be inserted into the base body (110) along a direction of insertion (B) that extends across the feed direction.

2. The discharge device according to claim 1, wherein the adapter (400) comprises a main section (401) with a passage opening for the piston rod (200), a fastening structure (402) on the main section (401) for the container (300) and two opposing retaining wings (403, 404) that extend in opposite lateral directions, and wherein the base body (110) comprises an insertion opening (141), which is complementary to the adapter (400), with two opposite slits (142, 143) that extend across the feed direction and across the direction of insertion, for accommodating the retaining wings (403, 404).

3. A discharge device comprising a base body (110), an operating lever (130) that is manually pivotable relative to the base body (110), and a piston rod (200) that is slidable relative to the base body (110), wherein the piston rod (200) is coupled to the operating lever (130) in such a manner that operation of the operating lever (130) causes the piston rod (200) to advance in a distal feed direction (A), wherein the discharge device comprises an adapter (400) that is formed separately from the base body (110) and is attached to the base body (110) and on which a fastening structure is formed for attachment of a container (300) substantially along a proximal direction (C) contrary to the feed direction (A), wherein the base body (110) comprises a housing and a separately manufactured retaining element (140) connected to the housing, wherein the adapter (400) is removably attached to the retaining element (140), **characterized in that** the retaining element (140) is configured to be slid into the housing along a fastening direction (D) that extends across the feed direction (A).

4. The discharge device according to any one of the preceding claims, wherein the adapter (400) is removable from the base body (110) while the piston rod (200) is held on the base body (110).

5. The discharge device according to any one of the preceding claims, wherein the adapter (400) comprises a screw connection or bayonet connection, which is open in the feed direction (A), for the container (300).

6. The discharge device according to any one of the preceding claims comprising a container (300), wherein the container (300) contains a highly viscous or semi-solid material that is preferably implantable, wherein the container (300) has the shape of a cylindrical pipe that along its entire length has a substantially constant cross-sectional area, wherein at each end of the pipe there is an external connecting structure for connection to the adapter, and wherein the connecting structures at each end are of the same design.

7. The discharge device according to claim 6, wherein the connecting structures at each end of the container (300) are substantially identical.

8. The discharge devivce according to claim 6 or 7, wherein the container (300) has the shape of a curved pipe (320; 330).

9. The discharge device according to any one of claims 6 to 8, wherein the container (300) is flexible in such a manner that its distal end is pivotable relative to the proximal end by at least 45°.

10. The discharge device according to any one of the preceding claims, wherein the piston rod (200) is flexible such that its distal end is pivotable along at least one bending direction by an angle of at least 45° relative to its proximal end.

11. The discharge device according to claim 10, wherein the piston rod (200) comprises a first side on which there is a plurality of teeth (211), wherein the operating lever (130) when operated exerts a feed force directly or indirectly on the teeth (211) in order to cause advancing of the piston rod (200), and wherein the piston rod (200) at least on a second side, which is opposite to the first side, comprises a plurality of incisions (212) in order to increase flexibility of the piston rod (200).

12. The discharge device according to claim 10 or 11 further comprising a container (300) in the form of a cylindrical pipe that is curved or that is flexible to such an extent that its distal end is pivotable on an angle (α) of at least 45° relative to the proximal end.

## Revendications

1. Dispositif distributeur, comportant un corps de base (110), un levier d'actionnement (130) manuellement pivotable par rapport au corps de base (110) et une tige de piston (200) pouvant être coulissée par rapport au corps de base (110), où la tige de piston (200) est couplée au levier d'actionnement (130) de telle sorte qu'un actionnement du levier d'actionnement (130) entraîne un avancement de la tige de piston (200) en direction distale d'avancement (A), où le dispositif distributeur comporte un adaptateur (400), qui est formé de manière séparée du corps de base (110) et qui est fixé sur le corps de base (110) et sur lequel une structure de fixation pour le montage d'un contenant (300) est formée sensiblement le long d'une direction proximale (C) contraire à une direction d'avancement (A), **caractérisé en ce que** l'adaptateur (400) peut être inséré dans le corps de base (110) le long d'une direction d'insertion (B) perpendiculaire à la direction d'avancement.

2. Dispositif distributeur selon la revendication 1, où l'adaptateur (400) comporte une section principale (401) ayant une ouverture traversante pour la tige de piston (200), une structure de fixation (402) pour le contenant (300) formée sur la section principale (401) ainsi que deux ailerons de fixation (403, 404) opposés et s'étendant en directions latérales contraires, et où le corps de base (110) comporte une ouverture d'insertion (141) complémentaire à l'adaptateur (400) ayant deux fentes (142, 143) opposées et s'étendant de manière perpendiculaire par rapport à la direction d'insertion et la direction d'avancement pour la réception des ailerons de fixation (403, 404).

3. Dispositif distributeur comportant un corps de base (110), un levier d'actionnement (130) manuellement pivotable par rapport au corps de base (110) et une tige de piston (200) pouvant être coulissée par rapport au corps de base (110), où la tige de piston (200) est couplée à la tige d'actionnement (130) de telle sorte qu'un actionnement du levier d'actionnement (130) entraîne un avancement de la tige de piston (200) en direction distale d'avancement (A), où le dispositif distributeur comporte un adaptateur (400), qui est formé de manière séparée du corps de base (110) et qui est monté sur le corps de base (110) et sur lequel une structure de fixation pour le montage d'un contenant (300) est formée sensiblement le long d'une direction proximale (C) contraire à la direction d'avancement (A), où le corps de base (110) comporte un boîtier ainsi qu'un élément de maintien (140) formé de manière séparée est relié au boîtier, où l'adaptateur (400) peut être fixé de manière amovible sur l'élément de maintien (140), **caractérisé en ce que** l'élément de maintien (140) peut être inséré dans le boîtier le long d'une direction de fixation (D) perpendiculaire à la direction d'avancement (A).

4. Dispositif distributeur selon une des revendications précédentes, où l'adaptateur (400) peut être détaché du corps de base (110), pendant que la tige de piston (200) est maintenue sur le corps de base (110).

5. Dispositif distributeur selon une des revendications précédentes, où l'adaptateur (400) comporte un raccordement vissé ou une fermeture à baïonnette pour le contenant (300) ouvert(e) en direction d'avancement (A).

6. Dispositif distributeur selon une des revendications précédentes, comportant un contenant (300), où le contenant (300) est rempli d'un matériau à haute viscosité ou semi-solide, préférablement pouvant être implanté, où le contenant (300) présente la forme d'un tuyau cylindrique, lequel présente une section transversale sensiblement constante le long de sa longueur complète, où une structure de raccordement pour le raccordement à l'adaptateur est formée aux deux extrémités et respectivement sur le côté extérieur, et où les structures de raccordement sont formées de manière identique aux deux extrémités.

7. Dispositif distributeur selon la revendication 6, où les structures de raccordement aux deux extrémités du contenant (300) sont sensiblement identiques.

8. Dispositif distributeur selon la revendication 6 ou 7, où le contenant (300) présente la forme d'un tuyau courbé (320; 330).

9. Dispositif distributeur selon une des revendications 6 à 8, où le contenant (300) est souple de telle sorte que son extrémité distale peut être pivotée par rapport à son extrémité proximale d'au moins 45°.

10. Dispositif distributeur selon une des revendications précédentes, où la tige de piston (200) est souple de telle sorte que son extrémité distale peut être pivotée par rapport à son extrémité proximale d'un angle d'au moins 45° le long d'au moins une direction de flexion.

11. Dispositif distributeur selon la revendication 10, où la tige de piston (200) comporte un premier côté, sur lequel une pluralité de dents (211) sont formées, où la tige d'actionnement (130) exerce une force d'avancement sur les dents (211) de manière directe ou indirecte lors de son actionnement, pour entraîner un avancement de la tige de piston (200), et où la tige de piston (200) comporte au moins sur un deuxième côté opposé au premier côté une pluralité d'incisions (212) pour augmenter la souplesse de la tige de piston (200).

12. Dispositif distributeur selon la revendication 10 ou 11, le quel comprend en outre un contenant (300) en forme d'un tuyau cylindrique, lequel est courbé ou lequel est souple de telle sorte que son extrémité distale peut être pivotée par rapport à l'extrémité proximale d'au moins 45°.
